# EUROPEAN PATENT SPECIFICATION

(11) **EP 2 536 424 B1**
(45) Date of publication and mention of the grant of the patent: **04.06.2014**
(21) Application number: 11713878.4
(22) Date of filing: 15.02.2011
(51) Int. Cl.: A61K 38/16, A61P 25/00

(54) **USE OF RHO GTPASE ACTIVATING TOXINS FOR THE TREATMENT AND/OR PREVENTION OF SYMPTOMATOLOGY ASSOCIATED WITH RETT SYNDROME**
VERWENDUNG VON RHO GTPASE-AKTIVIERENDES TOXINE ZUR BEHANDLUNG UND / ODER ZUR VORBEUGUNG VON RETT-SYNDROM-SYMPTOMATOLOGIE
UTILISATION DE TOXINES ACTIVANT LA RHO GTPASE POUR LE TRAITEMENT ET / OU LA PREVENTION DE SYMPTOMATOLOGIE ASSOCIÉE AU SYNDROME DE RETT

(30) Priority: 17.02.2010 IT RM20100063
(43) Date of publication of application: 26.12.2012
(73) Proprietor: Istituto Superiore di Sanità, 00161 Roma (IT)
(72) Inventor: FIORENTINI, Carla, I-00161 Roma (RM) (IT); LAVIOLA, Giovanni, I-00161 Roma (RM) (IT); RICCERI, Laura, I-00161 Roma (RM) (IT); DE FILIPPIS, Bianca, I-00161 Roma (RM) (IT); FABBRI, Alessia, I-00161 Roma (RM) (IT)
(74) Representative: Gitto, Serena
(86) International application number: PCT/IT2011/000039
(87) International publication number: WO 2011/101881

(56) References cited:
- EP-A2- 1 570 856
- WO-A1-2005/078099
- WO-A1-2007/115578
- WO-A2-2006/105998
- WO-A2-2007/017914
- DIANA GIOVANNI ET AL: "Enhancement of learning and memory after activation of cerebral Rho GTPases", PROCEEDINGS OF THE NATIONAL ACADEMY OF SCIENCES OF THE UNITED STATES OF AMERICA, vol. 104, no. 2, January 2007 (2007-01), pages 636-641, XP002599287, ISSN: 0027-8424
- DATABASE WPI Week 200645 Thomson Scientific, London, GB; AN 2006-445164 XP002599288, & WO 2006/051636 A1 (UNIV KURUME) 18 May 2006 (2006-05-18)
- KNUST Z ET AL: "Cytotoxic necrotizing factors (CNFs)-a growing toxin family", TOXINS 2010 MDPI AG CHE LNKD- DOI:10.3390/TOXINS2010116, vol. 2, no. 1, January 2010 (2010-01), pages 116-127, XP000002651665, ISSN: 2072-6651

### SEQUENCE LISTING

<110> Istituto Superiore di Sanità
<120> use of Rho GTPase activating toxines for the treatment and/or prevention of symptomatology associated with RETT syndrome
<130> PCT28102
<150> RM2010A000063
   <151> 2010-02-17
<160> 1
<170> PatentIn version 3.5
<210> 1
   <211> 11
   <212> PRT
   <213> Human immunodeficiency virus type 1
<400> 1
   Tyr Gly Arg Lys Lys Arg Arg Gln Arg Arg Arg
   1               5                   10

## Description

The present invention concerns the use of Rho GTPase activating toxines for the treatment and/or prevention of symptomatology associated with Rett syndrome (RTT). Particularly, the invention concerns the use of a toxin selected from the group consisting of: type 1 (CNF1), type 2 (CNF2) or type 3 (CNF3) Cytotoxic Necrotizing Factor from Escherichia coli or CNFY from Yersinia spp. or DNT from Bordetella spp, active fragments or mixtures thereof for the preparation of a medicament for the treatment and/or prevention of symptomatology associated with Rett syndrome (RTT). Said toxins being highly homologous at least as to catalytic portion.

Rett syndrome Rett (RTT) is a pathology of nervous system development affecting mostly feminine sex with 1/10.000 alive born incidence. Mutations of the gene encoding for MECP2 protein have been observed in more than 90% of the diagnosed cases along with a classic syndrome variant (Amir et al, 1999, Chahrour and Zoghbi, 2007). Although this protein would seem to exert a multifunctional role, the same acts preferentially as transcriptional repressor (Chahrour et al, 2008, Ogier and Katz, 2008). The location of MECP2 gene on X chromosome and the dominant inheritance mode, result in the fact that this pathology mostly affects feminine sex. The survival probability for male hemizygotes, as well as for female homozygotes, is in fact quite low.

Being included, according to DSM-IV, in Developmental Pervasive Disorders (Disturbi Pervasivi dello Sviluppo), RTT syndrome displays an extremely complex and invalidating symptomatology. An essential feature thereof is a prenatal and perinatal development with an apparently normal life up to approximately 6-18 months, followed by a regression period characterized by a progressive loss of both communicative and motor abilities and a retardation of cranial circumference growth usually resulting in microcephaly. At the end of this regression period, that in some cases is for several years, the development of RTT patients reaches a plateau associated to a great variety of specific symptoms. These include hand stereotyped movements, respiratory disorders, ECG anomalies, sleep disturbances, scoliosis, swellings, feeding disorders. During this phase autistic features, absence of movement coordination and remarkable mental retardation are also apparent. According to age progression the patients display a remarkable worsening of motor abilities. Life expectancy is extremely variable, several subjects reaching also 70 years of age (Mount et al, 2001, Hagberg, 2002).

The use of genetic engineering techniques allowed the generation of various transgenic murine models for RTT (Chen et al, 2001, Guy et al, 2001). The progression of the symptoms for these models follows a trend similar to that observable in RTT patients, as the appearance of apparent symptoms occurs only following to an apparently normal phase.

According to one RTT murine model, i.e. that comprising Mecp2 truncated gene (Mecp2-308) (Shahbazian et al 2002), a mutation associated to a less severe phenotype occurring in approximately 10% of RTT patients (Chahrour and Zoghbi, 2007), a detailed characterization of the phenotype has been carried out allowing during clearly symptomatic phase (4-10 age months) the presence of motor coordination difficulties, increased behaviour related to anxiety and cognitive deficit condition to be pointed out (Shahbazian et al, 2002, Moretti et al, 2005, Moretti et al, 2006; De Filippis et al, 2009; McGill et al, 2006).

Among RTT characteristic neuropathologic features: a selective alteration of dendrite and pyramidal neuron dimensions in frontal, motor and temporal regions; a dendritic spine decrease in some cerebral areas; at synaptic level and inter-neuron alterations, immunoreactivity alterations of MAP-2, a dendritogenesis marker, in some neurons populations (Armstrong, 2001) are reported. Post-mortem analysis of RTT patient brain further allowed to evidence that the presence of a reduced cerebral volume, rather than neuron number reduction, results from a marked decrease of neuronal soma dimensions and dendrite extension (Armstrong, 2005). Many of the evidences obtained by means of post-mortem studies on RTT patients, and particularly the presence of neuro-morphological anomalies, have been successively confirmed also for murine RTT available models (Fukuda et al, 2005; Belinchenko et al 2009). All together these evidences suggest that RTT is not a neurodegenerative disorder, the formation of the dendrites and axons could be preferentially affected by this syndrome and synaptic disorganization and a reduced plasticity could be responsible at least of some behavioural and motor anomalies associated to this disorder.

In one murine available model, it has been recently observed that Mecp2 gene activation in an advanced pathology phase is suitable to reverse at least some (mainly motor ones) characteristic RTT symptoms (Giacometti et al, 2007, Guy et al, 2007). Although the gene therapy approach does not represent a current clinically usable tool, these results demonstrate that the damage resulting from Mecp2 absence during early growth phases is not irreversible, suggesting therefore that this disabilitating syndrome can be pharmacologically successfully treated.

In the light of above it is therefore apparent the need to provide for new methods for the treatment of RTT symptomatology overcoming the disadvantages of known art methods.

The toxin named type 1 (CNF1) Cytotoxic Necrotizing Factor is a 110 Kda protein encoded by a gene occurring within chromosome of various Escherichia coli strains, that selectively and permanently activate Rho family GTPases and in addition is suitable to protect the cells against degenerative processes (apoptosis). CNF1 protein is able, due to N-terminal domain thereof, to penetrate cell membranes by endocytosis (Lemichez et al, 1997, Contamin et al, 2000) and activate Rho GTPases (in particular three Rho, Rac and Cdc42 sub-families) through deamidation of some critical glutamines by C-terminal domain (Flatau et al, 1997, Schmidt et al, 1997). The catalytic activity is shared by Escherichia coli CNF2 and Bordetella spp. DNT having high homology to CNF1.

Rho GTPases are ubiquitary proteins expressed in eukaryotic cells and act as switches in various signal transmission types. Particularly, Rho GTPases are involved in cytoskeleton actin re-modelling (Fukazawa et al, 2003). In effects, in neurons, the axon, dendrite and spine morphogenesis is just under the control of proteins belonging to Rho family (Etienne-Manneville & Hall, 2002).

The authors of the present invention now have administered, by intracerebroventricular route, CNF1 to one murine RTT available model (Mecp2-308 mouse) during a totally symptomatic phase (10 months of age) in order to test the possible effectiveness of CNF1 against at least some of the characteristic RTT symptoms. Particularly, according to this evaluation analysis of locomotor abilities (handling of nursery items and motor activity levels displayed during 24 hours), behaviours related to anxiety condition (time spent in an arena centre) and cognitive abilities (novel object recognition test and fear conditioning test) have been considered.

The Authors of the present invention have surprisingly found that the treatment with CNF1 remarkably improves motor coordination of mutant mouse as it contrasts the inability of Mecp2-308 mouse in handling nursery items. Such deficit in fact had already been evidenced in this RTT murine model and is considered like assimilable to hand apraxia often observable in RTT patients.

Also anomalies as to levels of locomotor activity displayed in the course of 24 hours by Mecp2-308 mouse and particularly the hypo-activity in active dark phase of the cycle have been effectively contrasted by treatment with CNF1. In addition to beneficial effects on the motor abilities, CNF1 also resulted in a remarkable improvement of cognitive abilities for Mecp2-308 mouse, as evaluated according to fear-conditioning test. The results obtained in this study suggest therefore that some characteristic symptoms of this syndrome can be ameliorated by pharmacological manipulation of the neuronal connectivity and identify cytoskeleton as possible target in order RTT phenotype to be improved.

It is therefore a specific object of the present invention a Rho GTPase activating toxin, said toxin being selected from the group consisting of dermonecrotizing toxin family, fragments thereof maintaining RhoGTPAse catalytic activity or mixtures of said toxins and/or fragments for use in the treatment and/or prevention of Rett syndrome (RTT) associated symptomatology. In particular, said toxins can be selected from type 1 (CNF1), type 2 (CNF2) or type 3 (CNF3) Cytotoxic Necrotizing Factor from Escherichia coli or CNFY from Yersinia spp. or DNT from Bordetella spp, and said fragments active on Rho GTPases can comprise or consist of C-terminal catalytic portion of CNF1 (CAA50007 genBank), CNF2 (AAA18229 genBank), DNT (AAA20995 genBank), CNF3 (AM263062.1 genBank) or CNFY (AF324349.1 genBank). In particular, C-terminal catalytic portion of CNF1 (CAA50007 genBank), CNF2 (AAA18229 genBank) comprises or consists of the peptide sequence from amino acid 720 to amino acid 1014 of said toxins, 866 cysteine and 881 histidine being essential amino acids for activity.

Toxins or fragments thereof according to the invention can be fused to short peptides, conjugated with nano-particles or carried by immunovectors or by a protein transduction domain, for example HIV-1 Tat peptide of 11 aa, i.e. YGRKKRRQRRR (SEQ ID NO: 1) which favours transmembrane transport and hematoencephalic barrier penetration.

The dose of said toxins, fragments or mixtures thereof can be from 0.0001 fmol/kg to 1 µmol/kg, preferably from 0.001 fmol/kg to 1 µmol/kg of patient body weight.

The medicament can be prepared in a form suitable to be administered to central nervous system, for example as a nasal spray form or an injectable form administrable by intracerebroventricolar or peripheral route, for example oral, subcutaneous, intramuscular, intravenous, sublingual, ocular.

According to a particular aspect of the present invention said toxins, active fragments or mixtures of said toxins and/or fragments thereof can be associated to one or more active principles for the symptomatic treatment of Rett syndrome, as for example anticonvulsant like for example topiramate, levetiracetam, carbamazepine, valproate. Topiramate is an anticonvulsant activating GABA dependent chlorine channels and at the same time inhibits excitatory neurotransmission,by means of a direct action on kainic acid and GluR5 receptors. Other active principles for symptomatic treatment of Rett syndrome are atypical antipsychotics as for example, olanzepine, risperidone which is an antagonist of 5ht2a and 5ht2c serotoninergic and d2 dopamine receptors, serotonine re-uptake inhibitors as for example fluoxetine, sertraline, citalopram, escitalopram, fluvoxamine, paroxetine, or Rho GTPase modulating compounds as for example, Clostrydium botulinum C3 transferase, ROCK kinase inhibitors (as for example Y27632), PAK inhibiting peptides, lisophosphatidic acid.

The symptomatology to be treated according to the present invention can be referred to patients in all the phases of Rett syndrome. In fact the disease phases have been classified as following: 1^{st} stage: initial disease phase (from 6 months to 1 and an half year); 2^{nd} stage: fast destructive or regression phase (from 1 to 4 years); 3^{rd} stage: stabilization phase (from prescholar to adult age); 4^{th} stage: late motor deterioration phase (variable age). Moreover it is possible the invention to be used also during so-called pre-symptomatic phase preceding the first phase of the syndrome.

It is a further object of the present invention a combination of one or more Rho GTPase activating toxins, said toxins being selected from dermonecrotizing toxin family, fragments thereof maintaining Rho GTPase catalytic activity or mixtures thereof, with one or more active principles for the symptomatic treatment of Rett syndrome for simultaneous, separated or sequential use in the treatment and/or prevention of symptomatology associated to Rett syndrome (RTT). Said one or more toxins can be selected from the group consisting of type 1 (CNF1), type 2 (CNF2) or type 3 (CNF3) Cytotoxic Necrotizing Factor from Escherichia coli or CNFY from Yersinia spp. or DNT from Bordetella spp; active fragments can be selected from C-terminal catalytic portions of CNF1 (CAA50007 genBank), CNF2 (AAA18229 genBank), DNT (AAA20995 genBank), CNF3 (AM263062.1 genBank) or CNFY (AF324349.1 genBank); and the active principles for symptomatic treatment of Rett syndrome can be selected from the group consisting of anticonvulsants, atypical antipsychotics, serotonine re-uptake inhibitors or Rho GTPase modulating compounds. Among Rho GTPase modulating compounds, Clostrydium botulinum C3 transferase, ROCK kinase inhibitors, PAK inhibiting peptides, lisophosphatidic acid can be mentioned.

The present invention now will be described by an illustrative, but not limitative way, according to preferred embodiments thereof with particular reference to enclosed drawings, wherein:
Figure 1 shows spontaneous locomotor activity in cage free mice a month after icv administration. Data is expressed as average ± SEM. * p<,05, ** p< .01; N=6-8
Figure 2 shows the effect of treatment with CNF1 on nest construction abilities 20 days after icv administration. Data is expressed as average ± SEM. * p<,05, ** p< .01.; N=7-9.
Figure 3 shows the modulation of behavioural response to a contextual fear conditioning test following treatment with CNF1, 12 days after icv administration. Data is expressed as average ± SEM * p<,05, ** p< .01; N=7-9.

### Example 1: Study of CNF1 effectiveness on Mecp2-308 mice

### MATERIALS AND METHODS

### Preparation of CNF1

CNF1 has been obtained from 392 ISS strain (kindly supplied by V. Falbo, Rome, Italy) and purified as previously described according to (Falzano et al, 1993). CNF1 C866S recombinant protein has been used as a control. In fact, the enzymatic activity of this protein on the Rho GTPases is eliminated by replacing cysteine at 866 position with serine (Schmidt et al, 1998). The plasmid encoding for CNF1 C866S has been kindly supplied by E. Lemichez (U627 INSERM, Nice, France) and the recombinant protein has been purified as described according to (Falzano et al, 1993).

### Intracerebroventricular administration of CNF1 (icv)

Following to total anesthesia (Equithesin, 3 ml/kg IP), mice have been immobilized in a stereotaxic apparatus. A solution of CNF1 (1 µl) or CNF1 C886S recombinant protein (1 µl) then is administered by intracerebroventricular route for approximately one minute according to the following stereotaxic coordinates AP=0.0; ML=+0.72 DV=-2.0 relatively to the bregma. After the operation the animals have been sutured with appropriate glue and left undisturbed on an heated small carpet to allow an adequate body temperature to be restored.

### Animals

As experimental subjects we have used Mecp2-308 mice of approximately 10 months of age and wild-type siblings, resulting from mating of female heterozygotes due to Mecp2 truncated mutation and male hemizygotes, from Jackson Laboratories Jackson (USA) (B6.129S-Mecp2tm1 Hzo/J, stock number: 005439; crossbred with C57BL/6J mice for at least 12 generations).

After icv administration, mice have been maintained isolated within transparent cages (33 × 13 × 14 cm) with sawdust on the bottom. Drinking water and a complete rodent specific diet have been provided ad libitum (Rieper, BZ, Italy). In the room where the animals were stabulated the light/dark cycle followed the following schedule: lights were automatically off/on at 8 a.m. and p.m., respectively.-Room temperature and relative humidity were constantly about 21 ± 1°C and about 60 ± 10%, respectively. All the adopted procedures are according to European Guidelines (86/609/EEC) and approved of by Italian Council of Ministers.

### Spontaneous locomotor activity

During immediately icv successive days and one month after the operation, the spontaneous locomotor activities within the cages of the mutants and controls have been evaluated using a software monitoring the animal movement by means of infrared sensors located on the top of the cages [ACTIVISCOPE system, NEWBEHAVIOR, CH; Internet site: www.newbehavior.com, see also (Dell'Omo et al., 2002, Adriani et al., 2003).] The profile of locomotor activity in the course of the 24 hours has been therefore calculated as average of 7 day measurements for each animal.

### Nest construction ability

Five days after the fear conditioning test (see below), in order to evaluate the motor coordination of the front limbs, the mutants and controls have been subjected to a test taking advantage of natural tendency of murine species to use paper material for nest construction. All the animals therefore have been supplied with a piece of paper (10 x 12 cm) and at 1, 48 and 72 hours, the nest position and quality have been estimated by means of an evaluating scale involving a zero score when paper has not been touched and 4 score when a nest have been well placed and constructed. About the application of this test see also (Moretti et al, 2005).

### Contextual and cued fear conditioning tests

Four days after the novel object recognition test (see below), the animals have been subjected to a fear conditioning test as described according to (Moretti et al, 2005). This test consists of 3 stages. During the training stage, the animals have been introduced in a room whose floor is constituted of metallic grate and can impart an electrical shock to the animal (Coulbourn Instruments, Allentown, USA). After standing undisturbed for 3 minutes in this room, the animals have been exposed to a sound for 30 seconds, in combination with an electrical shock in last 2 seconds (0,6 mA) (training). After additional 30 sec, the animals have been brought back to their cages. During the successive phase, after 24 hours, the animals have been re-located inside of the same cage wherein they had received in preceding day electrical shock and behaviour thereof has been observed for a total of 4 minutes (context test). The third and last phase has been carried out at least 1 hour after the second one. During this phase, the animal has been exposed to a new context and after 3 minutes during which it has been in condition to explore in undisturbed way the new environment, the animal has been again exposed to a tone similar to that heard in the course of training stage, but lasting 3 minutes and this time without electrical shock (tone test). The duration and frequency of the following behaviours then have been estimated in all the test stages: Crossing (passage number through two lines subdividing the floor in 3 equal parts), Rearing (raising of the body front portion and head), Wall rearing (raising of the body front portion from the floor using like as a support the cage walls, Grooming (body auto-cleaning), Inactivity (immobility, with the exception of little head movements).

### Novel object recognition test

A week after icv administration the animals have been evaluated according to a test for recognizing a new object as described in (Papaleo et al, 2008). At the beginning of the test the animals have been placed in an empty arena (40 × 40 cm) and left therein for approximately one hour in order the environment to be familiar (familiarization stage).

The day after the mice have been re-located within the same arena wherein two identical objects were present in addition. The duration of this phase was 10 minutes. After approximately an hour, the animals have been exposed again for 5 minutes within the same arena wherein one of the two familiar objects had been replaced with a new one. The time used by the animals in exploring the familiar and new object during the third phase, correspond, for the animals, to an index of recognizing the new object with respect to familiar one.

### Data analysis

Variance analysis (ANOVA) has been carried out on all the data. In particular, the used model considered like factor among the subjects the genotype and the treatment (CNF1 or CNF C886S) and one or more factors within subjects. Post-hoc comparisons have been carried out using Tukey test, that can be used in absence of significant results from ANOVA (Wilcox, 1987). Mann-Whitney test has been used for evaluation of the latencies that are not according to a normal distribution.

### RESULT AND DISCUSSION

### Spontaneous locomotor activity

Obtained results pointed out a remarkable effect of light and dark phases [F (1, 16) = 66.72; p < .001]. As expected, all the animals irrespective of genotype behaved more active during the dark rather than light stage. Generally, however, mutant mice behaved meaningfully less active than controls [F (1, 16) = 8.93; p = .009]. Post-hoc comparisons carried out for phase genotype interaction [F (1, 16) = 12.91; p =,024] have revealed further that the hypo-activity of the mutants was apparent only during the dark/active phase of the cycle (p <,01). During the week after the icv administration, the treatment with CNF1 did not affected in any way spontaneous locomotor activity.

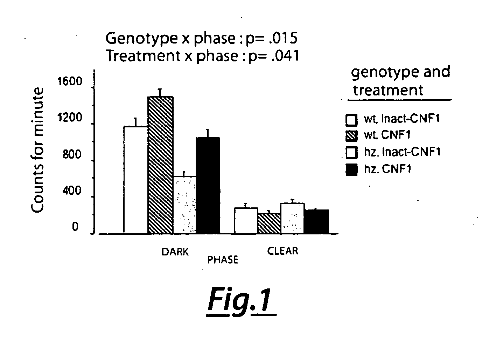

One month after the icv administration, in a similar way as already previously observed, the mutant mice behaved hypo-active during the dark phase of the cycle in comparison to the controls: an effect of light and dark phases [F (1,24) = 62.41; p <,001 and a phase genotype interaction [F (1,24) = 6.84; p =,015] have been again found (p <,05, after post-hoc comparisons; see Fig. 1).

Anyway, in contrast to what previously observed, one month after the icv administration, the treatment with CNF1 has meaningfully increased the levels of locomotor activity during the dark phase of cycle (p <,01, after post-hoc comparisons [F (1,24) = 4.63; p =,041]), thus the levels of activity of the mutants were again comparable to controls (see Fig. 1).

### Nest construction ability

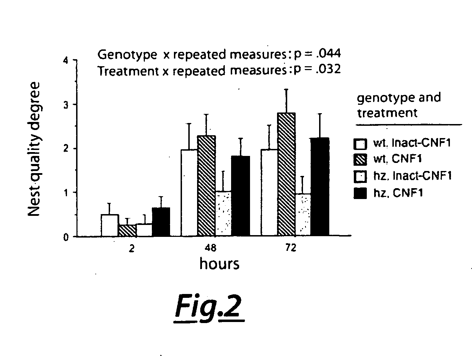

As expected all the animals irrespective of the experimental group improved nest quality over the time [F (2,52) = 40,64, P <,001]. However, the obtained results allowed a genotype and time elapsed after paper introduction interaction to be pointed out [F (2,52) = 3,32, p =,044]. Particularly, although a similar trend was already apparent 42 hours after the test start, the mutant mouse obtained scores significantly lower for nest evaluation 72 hours after the paper introduction into cages (p <,05, after post-hoc comparison), (see Fig. 2).

The treatment with CNF1 remarkably improved the motor abilities of the mutants increasing the score for nest evaluation test [p <,01, as a result of interaction treatment comparisons relative to time elapsed after paper introduction [F (2,52) = 3,67, p =,032)] see Fig. 2).

### Contextual and cued fear conditioning tests

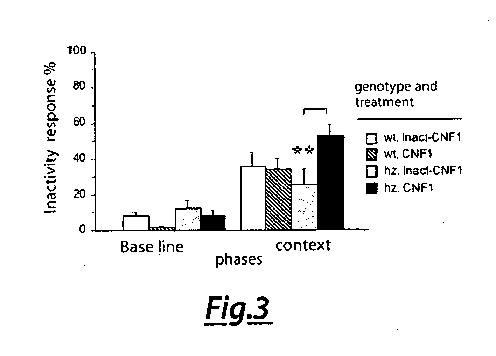

Compared to control values, all the experimental groups remarkably increased inactivity levels during both the context and tone test, thus demonstrating to remember both the context and the sound they had been exposed to in the preceding day during the training phase [Effect of the phase: Contextual: F (1, 26) = 54.57; p < .001; Cued: F (1, 26) = 47.57; p < .001]. The treatment with CNF1 remarkably increased the conditioned response, but only during the contextual fear conditioning test [p < the 0,05 as a result of post-hoc comparisons on the phase treatment interaction F (1, 26) = 5,02, p =,034]. In effects, post-hoc comparisons on the treatment genotype interaction for phase [p<,01, F (1,26) = 2.82; p =,10] allowed to point out that during the contextual fear conditioning test, CNF1 has remarkably improved performances only for mutant mouse, without influencing the control behaviour (see Fig. 3).

Also the locomotor activity, as measured like crossing frequency, displayed a remarkable decrease during test phases [Contextual: F (1, 26) = 13.79; p = .001; Cued: F (1, 22) = 32.63; p < .001]. In the course of tone test it has been moreover evidenced a genotype effect, because during this phase the mutant mouse behaved remarkably less active than the controls, a lower crossing number being carried out [F (1, 22) = 7,07, p <,014] (data not shown). On the other hand treatment effects during the phase of the cued fear conditioning test have not been evidenced.

### Novel object recognition test

During the familiarization phase, it has not been possible to evidence genotype effects on the locomotor activity, although some anomalies as to behavioural profile of the mutants, according to previously reported data, have been detected (De Filippis et al, 2009). Particularly, the mutants have passed less time within the arena central area [F (1,27) = 15,92, p <,001], confirming the presence in this murine model of increased behaviours related to anxiety condition. (McGill et al, 2006, De Filippis et al, 2009). In addition the mutants have displayed decreased levels for Rearing and Wall Rearing tests. [Genotype effect: F (1,27) = 4,81, p = .037; F (1,27) = 3,35, p =,071, respectively].

It has not been possible to evidence any genotype effect as to time passed by the animals in exploring the objects.

No effect of CNF1 treatment has been evidenced in this test.

### BIBLIOGRAPHY

Adriani W, Caprioli A, Granstrem O, Carli M, Laviola G (The spontaneously hypertensive-rat as an animal model of ADHD: evidence for impulsive and non-impulsive subpopulations. Neurosci Biobehav Rev 27:639-651.2003).
Amir RE, Van den Veyver IB, Wan M, Tran CQ, Francke U, Zoghbi HY (Rett syndrome is caused by mutations in X-linked MECP2, encoding methyl-CpG-binding protein 2. Nat Genet 23:185-188.1999).
Armstrong DD (Rett syndrome neuropathology review 2000. Brain Dev 23 Suppl 1:S72-76.2001).
Armstrong DD (Neuropathology of Rett syndrome. J Child Neurol 20:747-753.2005).
Belichenko PV, Wright EE, Belichenko NP, Masliah E, Li HH, Mobley WC, Francke U (Widespread changes in dendritic and axonal morphology in Mecp2-mutant mouse models of Rett syndrome: evidence for disruption of neuronal networks. J Comp Neurol 514:240-258.2009)
Birke LI, Sadler D (Differences in maternal behavior of rats and the sociosexual development of the offspring. Developmental psychobiology 20:85-99.1987).
Chahrour M, Jung SY, Shaw C, Zhou X, Wong ST, Qin J, Zoghbi HY (MeCP2, a key contributor to neurological disease, activates and represses transcription. Science 320:1224-1229.2008).
Chahrour M, Zoghbi HY (The story of Rett syndrome: from clinic to neurobiology. Neuron 56:422-437.2007).
Chen RZ, Akbarian S, Tudor M, Jaenisch R (Deficiency of methyl-CpG binding protein-2 in CNS neurons results in a Rett-like phenotype in mice. Nat Genet 27:327-331.2001).
Contamin S, Galmiche A, Doye A, Flatau G, Benmerah A, Boquet P (The p21 Rho-activating toxin cytotoxic necrotizing factor 1 is endocytosed by a clathrin-independent mechanism and enters the cytosol by an acidic-dependent membrane translocation step. Mol Biol Cell 11:1775-1787.2000).
De Filippis B, Ricceri L, Laviola G (Early postnatal behavioral changes in the Mecp2-308 truncation mouse model of Rett syndrome. Genes, brain, and behavior.2009).
Dell'Omo G, Vannoni E, Vyssotski AL, Di Bari MA, Nonno R, Agrimi U, Lipp HP (Early behavioural changes in mice infected with BSE and scrapie: automated home cage monitoring reveals prion strain differences. The European journal of neuroscience 16:735-742.2002).
Etienne-Manneville S, Hall A (Rho GTPases in cell biology. Nature 420:629-635.2002)
Falzano L, Fiorentini C, Donelli G, Michel E, Kocks C, Cossart P, Cabanie L, Oswald E, Boquet P (Induction of phagocytic behaviour in human epithelial cells by Escherichia coli cytotoxic necrotizing factor type 1. Mol Microbiol 9:1247-1254.1993).
Flatau G, Lemichez E, Gauthier M, Chardin P, Paris S, Fiorentini C, Boquet P (Toxin-induced activation of the G protein p21 Rho by deamidation of glutamine. Nature 387:729-733.1997).
Fukazawa Y, Saitoh Y, Ozawa F, Ohta Y, Mizuno K, Inokuchi K (Hippocampal LTP is accompanied by enhanced F-actin content within the dendritic spine that is essential for late LTP maintenance in vivo. Neuron 38:447-460.2003).
Fukuda T, Itoh M, Ichikawa T, Washiyama K, Goto Y (Delayed maturation of neuronal architecture and synaptogenesis in cerebral cortex of Mecp2-deficient mice. J Neuropathol Exp Neurol 64:537-544.2005).
Giacometti E, Luikenhuis S, Beard C, Jaenisch R (Partial rescue of MeCP2 deficiency by postnatal activation of MeCP2. Proc Natl Acad Sci U S A 104:1931-1936.2007).
Guy J, Gan J, Selfridge J, Cobb S, Bird A (Reversal of neurological defects in a mouse model of Rett syndrome. Science 315:1143-1147.2007).
Guy J, Hendrich B, Holmes M, Martin JE, Bird A (A mouse Mecp2-null mutation causes neurological symptoms that mimic Rett syndrome. Nat Genet 27:322-326.2001).
Hagberg B (Clinical manifestations and stages of Rett syndrome. Ment Retard Dev Disabil Res Rev 8:61-65.2002).
Hoffbuhr KC, Moses LM, Jerdonek MA, Naidu S, Hoffman EP (Associations between MeCP2 mutations, X-chromosome inactivation, and phenotype. Ment Retard Dev Disabil Res Rev 8:99-105.2002).
Kaufmann WE, Johnston MV, Blue ME (MeCP2 expression and function during brain development: implications for Rett syndrome's pathogenesis and clinical evolution. Brain Dev 27 Suppl 1:S77-S87.2005).
Laviola G, Rea M, Morley-Fletcher S, Di Carlo S, Bacosi A, De Simone R, Bertini M, Pacifici R (Beneficial effects of enriched environment on adolescent rats from stressed pregnancies. The European journal of neuroscience 20:1655-1664.2004).
Laviola G, Sedowofia K, Innes J, Clayton R, Manning A (Genetic differences in maternal behaviour patterns in mice administered phenobarbital during pregnancy. Psychopharmacology 102:383-390.1990).
Laviola G, Terranova ML, Sedowofia K, Clayton R, Manning A (A mouse model of early social interactions after prenatal drug exposure: a genetic investigation. Psychopharmacology 113:388-394.1994).
Lemichez E, Flatau G, Bruzzone M, Boquet P, Gauthier M (Molecular localization of the Escherichia coli cytotoxic necrotizing factor CNF1 cell-binding and catalytic domains. Mol Microbiol 24:1061-1070.1997).
McGill BE, Bundle SF, Yaylaoglu MB, Carson JP, Thaller C, Zoghbi HY (Enhanced anxiety and stress-induced corticosterone release are associated with increased Crh expression in a mouse model of Rett syndrome. Proc Natl Acad Sci U S A 103:18267-18272.2006).
Moretti P, Bouwknecht JA, Teague R, Paylor R, Zoghbi HY (Abnormalities of social interactions and home-cage behavior in a mouse model of Rett syndrome. Hum Mol Genet 14:205-220.2005).
Moretti P, Levenson JM, Battaglia F, Atkinson R, Antalffy B, Amstrong D, Arancio O, Sweatt JD, Zoghbi HY (Learning and Memory and Synaptic Plasticity Are Impaired in a Mouse Model of Rett Syndrome. J Neurosci 26:319-327.2006).
Mount RH, Hastings RP, Reilly S, Cass H, Charman T (Behavioural and emotional features in Rett syndrome. Disabil Rehabil 23:129-138.2001).
Ogier M, Katz DM (Breathing dysfunction in Rett syndrome: Understanding epigenetic regulation of the respiratory network. Respir Physiol Neurobiol.2008).
Papaleo F, Crawley JN, Song J, Lipska BK, Pickel J, Weinberger DR, Chen J (Genetic dissection of the role of catechol-O-methyltransferase in cognition and stress reactivity in mice. J Neurosci 28:8709-8723.2008).
Ricceri L, De Filippis B, Laviola G (Mouse models of Rett syndrome: from behavioural phenotyping to preclinical evaluation of new therapeutic approaches. Behavioural pharmacology 19:501-517.2008). Schmidt G, Sehr P, Wilm M, Selzer J, Mann M, Aktories K (Gln 63 of Rho is deamidated by Escherichia coli cytotoxic necrotizing factor-1. Nature 387:725-729.1997).
Schmidt G, Selzer J, Lerm M, Aktories K (The Rho-deamidating cytotoxic necrotizing factor 1 from Escherichia coli possesses transglutaminase activity. Cysteine 866 and histidine 881 are essential for enzyme activity. The Journal of biological chemistry 273:13669-13674.1998).
Shahbazian M, Young J, Yuva-Paylor L, Spencer C, Antalffy B, Noebels J, Armstrong D, Paylor R, Zoghbi H (Mice with truncated MeCP2 recapitulate many Rett syndrome features and display hyperacetylation of histone H3. Neuron 35:243-254.2002).
Stearns NA, Schaevitz LR, Bowling H, Nag N, Berger UV, Berger-Sweeney J (Behavioral and anatomical abnormalities in Mecp2 mutant mice: a model for Rett syndrome. Neuroscience 146:907-921.2007).
Tudor M, Akbarian S, Chen RZ, Jaenisch R (Transcriptional profiling of a mouse model for Rett syndrome reveals subtle transcriptional changes in the brain. Proc Natl Acad Sci U S A 99:15536-15541.2002).
Wilcox RG (ed.) (1987) New statistical procedures for the social sciences. NJ.

## Claims

1. Rho GTPase activating toxin, said toxin being selected from dermonecrotizing toxin family, fragments thereof maintaining Rho GTPAse catalytic activity or mixtures of said toxins and/or fragments
for use in the treatment and/or prevention of Rett syndrome (RTT) associated symptomatology,
wherein said toxin is selected from type 1 (CNF1), type 2 (CNF2) or type 3 (CNF3) Cytotoxic Necrotizing Factor from Escherichia coli or CNFY from Yersinia spp. or DNT from Bordetella spp, and
said fragments active on Rho GTPases comprise or consist of C-terminal catalytic portion of CNF1 (CAA50007 genBank), CNF2 (AAA18229 genBank), DNT (AAA20995 genBank), CNF3 (AM263062.1 genBank) or CNFY (AF324349.1 genBank).

2. Toxin according to claim 1 for use according to claim 1, wherein said toxin or fragments thereof are fused to short peptides, conjugated to nano-particles or carried by immunovectors or by a protein transduction domain that favours transmembrane transport and hematoencephalic barrier penetration.

3. Toxin according to claim 2 for use according to claim 2, wherein the protein transduction domain is HIV-1 Tat 11 aa peptide of SEQ ID No. 1 having YGRKKRRQRRR sequence.

4. Toxin according to anyone of claims 1-3 for use according to anyone of claims 1-3, wherein the dose of said toxin or fragments or mixtures thereof is from 0.0001 fmol/kg to 1 µmol/kg, preferably from 0.001 fmol/kg to 1 µmol/kg of patient body weight.

5. Toxin according to anyone of preceding claims for use according to anyone of preceding claims wherein said toxin is the active principle of a medicament in a form suitable to be administered to central nervous system or according to peripheral route.

6. Toxin according to claim 5 for use according to claim 5 wherein said peripheral route is selected from oral, subcutaneous, intramuscular, intravenous, sublingual, ocular routes.

7. Toxin according to claim 5 for use according to claim 5 wherein said medicament is in nasal spray or intracerebroventricular route administrable form.

8. Toxin according to anyone of preceding claims for use according to anyone of preceding claims wherein said toxin, fragments thereof or mixtures of said toxins and/or fragments are associated to one or more active principles for the symptomatic treatment of Rett syndrome selected from the group consisting of anticonvulsants, atypical antipsychotics, serotonine re-uptake inhibitors or Rho GTPase modulating compounds, the latter being selected from the group consisting of Clostrydium botulinum C3 transferase, Y27632, lisophosphatidic acid.

9. Toxin according to anyone of claims from 1 to 8 for use according to anyone of claims from 1 to 8 wherein the symptomatology is referred to patients in all the phases of Rett syndrome.

10. Combination of one or more Rho GTPase activating toxins, said toxins being selected from dermonecrotizing toxin family, fragments thereof maintaining Rho GTPase catalytic activity or mixtures thereof, with one or more active principles for the symptomatic treatment of Rett syndrome
for simultaneous, separated or sequential use in the treatment and/or prevention of symptomatology associated to Rett syndrome (RTT), wherein
said one or more toxins are selected from the group consisting of type 1 (CNF1), type 2 (CNF2) or type 3 (CNF3) Cytotoxic Necrotizing Factor from Escherichia coli or CNFY from Yersinia spp. or DNT from Bordetella spp;
said active fragments are selected from C-terminal catalytic portions of CNF1 (CAA50007 genBank), CNF2 (AAA18229 genBank), DNT (AAA20995 genBank), CNF3 (AM263062.1 genBank) or CNFY (AF324349.1 genBank); and
said active principles for symptomatic treatment of Rett syndrome are selected from the group consisting of anticonvulsants, atypical antipsychotics, serotonine re-uptake inhibitors or Rho GTPase modulating compounds, the latter being selected from the group consisting of Clostrydium botulinum C3 transferase, Y27632, lisophosphatidic acid.

## Patentansprüche

1. Rho-GTPase aktivierendes Toxin, wobei das Toxin aus der Familie der dermonekrotisierenden Toxine, Fragmenten davon, welche die katalytische Aktivität gegenüber Rho-GTPase beibehalten haben, oder Mischungen der Toxine und/oder Fragmenten davon ausgewählt ist,
zur Verwendung bei der Behandlung und/oder Vorbeugung von mit dem Rett-Syndrom (RTT) assoziierter Symptomatik,
wobei das Toxin aus Typ 1 (CNF1), Typ 2 (CNF2) oder Typ 3 (CNF3) des cytotoxisch-nekrotisierenden Faktors (CNF) von Escherichia coli, CNFY von Yersinia spp. oder DNT von Bordetella spp. ausgewählt ist, und
wobei die gegenüber Rho-GTPasen aktiven Fragmente den C-terminalen katalytischen Abschnitt von CNF1 (CAA50007 genBank), CNF2 (AAA18229 genBank), DNT (AAA20995 genBank), CNF3 (AM263062.1 genBank) oder CNFY (AF324349.1 genBank) umfassen oder daraus bestehen.

2. Toxin nach Anspruch 1 zur Verwendung nach Anspruch 1, wobei das Toxin oder die Fragmente davon mit kurzen Peptiden fusioniert sind, mit Nanopartikeln konjugiert sind oder von Immunovektoren oder einer Proteintransduktionsdomäne, welche den Transmembrantransport und das Passieren der Blut-Hirn-Schranke fördert, getragen werden.

3. Toxin nach Anspruch 2 zur Verwendung nach Anspruch 2, wobei die Proteintransduktionsdomäne das HIV-1-Tat 11-aa-Peptid von SEQ-ID No. 1 mit der Sequenz YGRKKRRQRRR ist.

4. Toxin nach irgendeinem der Ansprüche 1 bis 3 zur Verwendung nach irgendeinem der Ansprüche 1 bis 3, wobei die Dosis des Toxins oder der Fragmente oder der Mischungen davon 0,0001 fmol/kg bis 1 µmol/kg, vorzugsweise 0,001 fmol/kg bis 1 µmol/kg Körpergewicht des Patienten beträgt.

5. Toxin nach irgendeinem der vorhergehenden Ansprüche zur Verwendung nach irgendeinem der vorhergehenden Ansprüche, wobei das Toxin den Wirkstoff eines Medikaments in einer Form, welche für die Verabreichung an das Zentralnervensystem oder über einen peripheren Verabreichungsweg geeignet ist, darstellt.

6. Toxin nach Anspruch 5 zur Verwendung nach Anspruch 5, wobei der periphere Verabreichungsweg aus oralen, subkutanen, intramuskulären, intravenösen, sublingualen und okularen Verabreichungswegen ausgewählt ist.

7. Toxin nach Anspruch 5 zur Verwendung nach Anspruch 5, wobei das Medikament in Form eines Nasensprays oder in einer auf dem intracerebroventrikulären Weg verabreichbaren Form vorliegt.

8. Toxin nach irgendeinem der vorhergehenden Ansprüche zur Verwendung nach irgendeinem der vorhergehenden Ansprüche, wobei das Toxin, die Fragmente davon oder Mischungen der Toxine und/oder Fragmente mit einem oder mehreren Wirkstoff(en) für die symptomatische Behandlung des Rett-Syndroms assoziiert ist/sind, welche(r) aus der Gruppe, bestehend aus Antikonvulsiva, atypischen Antipsychotika, Serotonin-Wiederaufnahmehemmern und Rho-GTPase modulierenden Verbindungen, wobei die letzteren aus der Gruppe, die aus Clostridium botulinum C3-Transferase, Y27632, Lysophosphatidinsäure besteht, ausgewählt sind, ausgewählt ist/sind.

9. Toxin nach irgendeinem der Ansprüche 1 bis 8 zur Verwendung nach irgendeinem der Ansprüche 1 bis 8, wobei die Symptomatik Patienten in allen Phasen des Rett-Syndroms betrifft.

10. Kombination von einem oder mehreren Rho-GTPase aktivierenden Toxin(en), wobei die Toxine aus der Familie der dermonekrotisierenden Toxine, Fragmenten davon, welche die katalytische Aktivität gegenüber Rho-GTPase beibehalten haben, oder Mischungen davon ausgewählt sind, mit einem oder mehreren Wirkstoff(en) für die symptomatische Behandlung des Rett-Syndroms,
für die gleichzeitige, separate oder sequentielle Verwendung bei der Behandlung und/oder Vorbeugung von mit dem Rett-Syndrom (RTT) assoziierter Symptomatik, wobei
das eine oder die mehreren Toxin(e) aus der Gruppe ausgewählt ist/sind, die aus Typ 1 (CNF1), Typ 2 (CNF2) oder Typ 3 (CNF3) des cytotoxisch-nekrotisierenden Faktors (CNF) von Escherichia coli, CNFY aus Yersinia spp. und DNT von Bordetella spp. besteht;
die aktiven Fragmente aus den C-terminalen katalytischen Abschnitten von CNF1 (CAA50007 genBank), CNF2 (AAA18229 genBank), DNT (AAA20995 genBank), CNF3 (AM263062.1 genBank) oder CNFY (AF324349.1 genBank) ausgewählt sind; und
die Wirkstoffe für die symptomatische Behandlung des Rett-Syndroms aus der Gruppe ausgewählt sind, die aus Antikonvulsiva, atypischen Antipsychotika, Serotonin-Wiederaufnahmehemmern und Rho-GTPase modulierenden Verbindungen besteht, wobei die letzteren aus der Gruppe, die aus Clostridium botulinum C3-Transferase, Y27632, Lysophosphatidinsäure besteht, ausgewählt sind.

## Revendications

1. Toxine activant la Rho GTPase, ladite toxine étant choisie parmi la famille des toxines dermonécrosantes, les fragments de celles-ci, maintenant l'activité catalytique de la Rho GTPase ou les mélanges desdites toxines et/ou desdits fragments
à utiliser dans le traitement et/ou la prévention de la symptomatologie associée au syndrome de Rett (RTT)
où ladite toxine est choisie parmi le Facteur Nécrosant Cytotoxique de type 1 (CNF1), de type 2 (CNF2) ou de type 3 (CNF3) d'*Escherichia coli* ou CNFY d'*Yersinia spp.* ou DNT de *Bordetella spp*., et
lesdits fragments actifs sur les Rho GTPases comprennent ou sont constitués d'une portion catalytique C-terminale de CNF1 (CAA50007 genBank), CNF2 (AAA18229 genBank), DNT (AAA20995 genBank), CNF3 (AM263062.1 genBank) ou CNFY (AF324349.1 genBank).

2. Toxine selon la revendication 1 à utiliser selon la revendication 1, où ladite toxine ou ses fragments sont fusionnés à des peptides courts, conjugués à des nanoparticules ou transportés par des immunovecteurs ou par un domaine de transduction de protéines qui favorise le transport transmembranaire et le passage à travers la barrière hématoencéphalique.

3. Toxine selon la revendication 2 à utiliser selon la revendication 2, où le domaine de transduction de protéines est le peptide Tat 11 aa du VIH-1 de SEQ ID No. 1 ayant la séquence YGRKKRRQRRR.

4. Toxine selon l'une quelconque des revendications 1-3 à utiliser selon l'une quelconque des revendications 1-3, où la dose de ladite toxine ou desdits fragments ou de leurs mélanges est de 0,0001 fmol/kg à 1 µmol/kg, de préférence, de 0,001 fmol/kg à 1 µmol/kg de poids corporel du patient.

5. Toxine selon l'une quelconque des revendications précédentes à utiliser selon l'une quelconque des revendications précédentes, où ladite toxine est le principe actif d'un médicament sous une forme appropriée pour être administrée dans le système nerveux central ou par une voie périphérique.

6. Toxine selon la revendication 5 à utiliser selon la revendication 5, où ladite voie périphérique est choisie parmi la voie orale, sous-cutanée, intramusculaire, intraveineuse, sublinguale, oculaire.

7. Toxine selon la revendication 5 à utiliser selon la revendication 5, où ledit médicament est sous forme de vaporisateur nasal ou sous forme administrable par voie intracérébroventriculaire.

8. Toxine selon l'une quelconque des revendications précédentes, à utiliser selon l'une quelconque des revendications précédentes, où ladite toxine, ses fragments ou les mélanges desdites toxines et/ou desdits fragments sont associés à un ou plusieurs principes actifs destinés au traitement symptomatique du syndrome de Rett, choisi(s) dans le groupe constitué par les anticonvulsivants, les antipsychotiques atypiques, les inhibiteurs de la réabsorption de la sérotonine ou les composés modulant la Rho GTPase, ces derniers étant choisis dans le groupe constitué par la C3 transférase de *Clostrydium botulinum*, Y27632, l'acide lisophosphatidique.

9. Toxine selon l'une quelconque des revendications 1 à 8, à utiliser selon l'une quelconque des revendications 1 à 8, où la symptomatologie concerne les patients dans toutes les phases du syndrome de Rett.

10. Combinaison d'une ou de plusieurs toxines activant la Rho GTPase, lesdites toxines étant choisies parmi la famille des toxines dermonécrosantes, les fragments de celles-ci, maintenant l'activité catalytique de la Rho GTPase ou leurs mélanges, avec un ou plusieurs principes actifs pour le traitement symptomatique du syndrome de Rett
pour une utilisation simultanée, séparée ou séquentielle dans le traitement et/ou la prévention de la symptomatologie associée au syndrome de Rett (RTT), où
ladite ou lesdites toxines sont choisies dans le groupe constitué par le Facteur Nécrosant Cytotoxique de type 1 (CNF1), de type 2 (CNF2) ou de type 3 (CNF3) *d'Escherichia coli* ou CNFY d'*Yersinia spp.* ou DNT de *Bordetella spp.;*
lesdits fragments actifs sont choisis parmi les portions catalytiques C-terminales de CNF1 (CAA50007 genBank), CNF2 (AAA18229 genBank), DNT (AAA20995 genBank), CNF3 (AM263062.1 genBank) ou CNFY (AF324349.1 genBank); et
lesdits principes actifs destinés au traitement symptomatique du syndrome de Rett sont choisis dans le groupe constitué par les anticonvulsivants, les antipsychotiques atypiques, les inhibiteurs de la réabsorption de la sérotonine ou les composés modulant la Rho GTPase, ces derniers étant choisis dans le groupe constitué par la C3 transférase de *Clostrydium botulinum*, Y27632, l'acide lisophosphatidique.
